# EUROPÄISCHE PATENTANMELDUNG

(11) **EP 1 287 798 A1**
(43) Veröffentlichungstag der Anmeldung: **05.03.2003**
(21) Anmeldenummer: 02018998.1
(22) Anmeldetag: 27.08.2002
(51) Int. Cl.: A61F 13/15

(54) **Vorrichtung zum Herstellen von Hygieneprodukten**

(30) Priorität: 04.09.2001 DE 10144028
(71) Anmelder: Winkler + Dünnebier Aktiengesellschaft, 56564 Neuwied (DE)
(72) Erfinder: Becker, Albert, 56581 Ehlscheid (DE)
(74) Vertreter: Schieferdecker, Lutz, Dipl.-Ing.

(57) **Zusammenfassung**

Die Erfindung betrifft eine Vorrichtung zum Herstellen von Hygieneprodukten und umfaßt mehrere Stationen 2 mit zumindest teilweise unterschiedlichen Maschinenkomponenten 3 sowie mit größtenteils eigenen Antrieben 4. Die Maschinenkomponenten 3 und die Antriebe 4 sind jeweils als Montageeinheiten auf Trageplatten 9 zusammengefaßt und in einem Maschinengestell befestigbar, das vertikale Stützen 18 und horizontal verbindende Streben 17, 19, 20 aufweist.

Der Kern der Erfindung besteht darin, daß die als Kleinmodule vorgesehen, aus Maschinenkomponenten 3 und Antrieben 4 sowie Trageplatten 4 bestehenden Montageeinheiten mindestens teilweise zu großen, ebenfalls Montageeinheiten bildenden Großmodulen 12 zusammengefaßt sind und daß die mit Kleinmodulen 10 bestückten Großmodule 12 zur Bildung der das Maschinengestell, die Maschinenkomponenten 3 und ihren Antrieb 4 umfassenden Vorrichtung 1 miteinander verbindbar sind (Fig. 1).

## Beschreibung

Die Erfindung betrifft eine Vorrichtung zum Herstellen von Hygieneprodukten und umfaßt gemäß den Merkmalen des Oberbegriffes von Patentanspruch 1 mehrere Stationen mit zumindest teilweise unterschiedlichen Maschinenkomponenten und überwiegend eigenen Antrieben. Charakteristisch für eine Vorrichtung der genannten Art ist ferner, daß die Maschinenkomponenten ganz oder teilweise und ihre Antriebe in Montageeinheiten auf Trageplatten zusammengefaßt und an einem Maschinengestell befestigbar sind, das vertikale Stützen und horizontal verbindende Streben aufweist.

Die Aufteilung der Maschinenkomponenten und der Antriebe in eine Vielzahl unterschiedlicher, jeweils auch Trageplatten umfassende Montageeinheiten weist verschiedene Vorteile auf. Die einzelnen Montageeinheiten können unabhängig voneinander hergestellt und dann in das als Träger für die Maschineneinheiten dienende Tragegestell integriert werden. Hiermit sind produktionstechnische Vorteile verbunden. Diese produktionstechnischen Vorteile lassen sich aber nur dann realisieren, wenn die Trageplatten genormt sind. Die Normung der Trageplatten ist schließlich nicht nur mit Vorteilen, sondern auch mit Nachteilen für das Maschinengestell verbunden, das aus einer großen Anzahl vertikaler Stützen und horizontal verbindender Streben auf einem aus Bodenplatten gebildeten Gestellboden besteht.

Vor allem die bei einem Notstop auftretenden dynamischen Kräfte in Verbindung mit den üblichen statischen Kräften sind erheblich und erfordern einen nicht unbeträchtlichen Aufwand für die Gestaltung des Maschinengestelles.

Der Erfindung liegt daher die Aufgabe zugrunde, optimale Bedingungen für die Montage und die Demontage der Vorrichtung und/oder von einzelnen Komponenten im Falle von Reparaturen oder bei notwendigen Umrüstarbeiten zu schaffen, ohne daß dadurch die notwendige Festigkeit und Steifigkeit des als Träger für die Maschinenkomponenten und die Antriebe dienenden Maschinengestelles verloren geht.

Zur Lösung dieser Aufgabe sieht die Erfindung mit den Merkmalen des kennzeichnenden Teiles von Patentanspruch 1 vor, daß die als Kleinmodule vorgesehenen, aus Maschinenkomponenten und Antrieben sowie Trageplatten bestehenden Montageeinheiten jeweils zu größeren, ebenfalls Montageeinheiten bildenden Großmodulen zusammengefaßt sind und daß die mit Kleinmodulen bestückten Großmodule zur Bildung der das Maschinengestell, die Maschinenkomponenten und die Antriebe umfassenden Vorrichtung miteinander verbindbar sind.

Erfindungsgemäß gehören somit auch Teile des Maschinengestelles jeweils zu einem Großmodul ebenso wie verschiedene Maschinenkomponenten und Antriebe. Jedes Großmodul umfaßt entsprechend ein Montagegerüst als Träger, wobei das Montagegerüst in gleicher Weise eine Tragefunktion besitzt wie die Trageplatten im Falle der Kleinmodule.

In Weiterbildung der Erfindung ist vorgesehen, daß das Montagegerüst des Großmoduls Rahmenelemente mit kurzen, horizontal liegenden Streben und langen, vertikal angeordneten Stützen sowie diese in Maschinenlängsrichtung verbindende Streben aufweist.

Weitere Merkmale der Erfindung gehen aus Unteransprüchen und der Beschreibung im Zusammenhang mit der Zeichnung hervor.

Die Erfindung wird nachstehend anhand von Ausführungsbeispielen die in der Zeichnung dargestellt sind, näher beschrieben. Dabei zeigen:
- Fig. 1: schematisch sowie zum Teil in auseinander gezogener Darstellung mehrere Kleinmodule und Großmodule einer Vorrichtung zum Herstellen von Hygieneprodukten;
- Fig. 2: in größerem Maßstab eine perspektivische Ansicht von drei Kleinmodulen bestehend aus Maschinenkomponenten und als Träger dienenden Trageplatten;
- Fig. 3: eine Ansicht eines als Träger eines Großmoduls dienenden Montagegerüstes in kleinerem Maßstab;
- Fig. 4: eine Draufsicht auf das Montagegerüst;
- Fig. 5.: eine perspektivische Ansicht des Montagegerüstes von vorne in nochmals kleinerem Maßstab;
- Fig. 6: eine perspektivische Ansicht des Montagegerüstes von hinten;
- Fig. 7: eine perspektivische Ansicht des Montagegerüstes wie in Fig. 5 zusammen mit einem Schaltschrank
- Fig. 8: eine Seitenansicht des Montagegerüstes mit Schaltschrank in kleinerem Maßstab und
- Fig. 9: eine perspektivische Ansicht wie in Fig. 7 mit zwei verschiedenen Montagegerüsten.

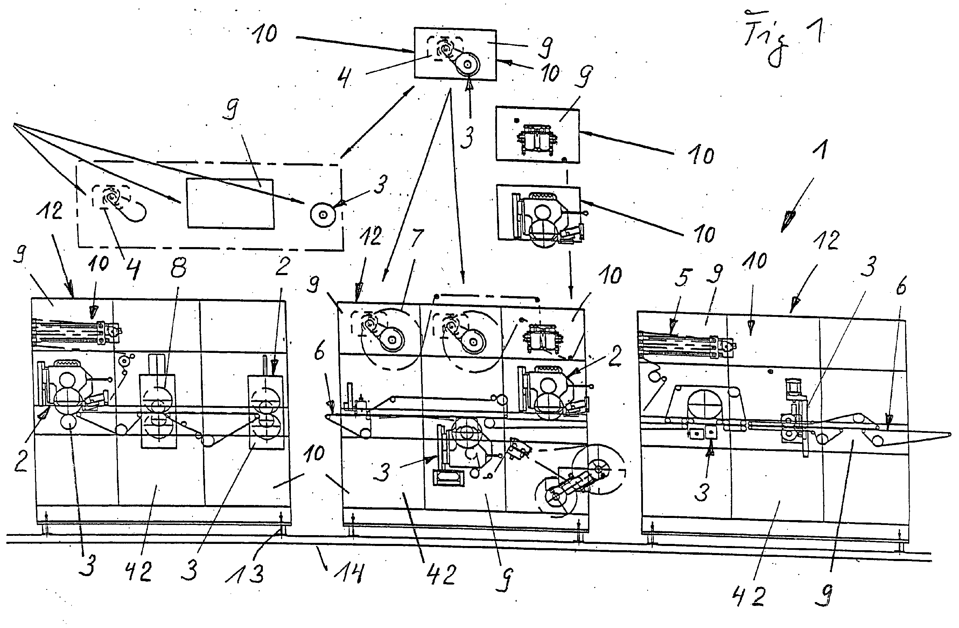

Eine in Fig. 1 nur zum Teil dargestellte Vorrichtung 1 zum Herstellen von Hygieneprodukten umfaßt je nach Bedarf mehrere Stationen 2 mit zumindest teilweise unterschiedlichen Maschinenkomponenten 3 sowie mit größtenteils eigenen Antrieben 4. Bei den Maschinenkomponenten 3 kann es sich um Speicherelemente 5, Transporteinrichtungen 6 , Abrolleinrichtungen 7 , Querschneider 8, Falteinrichtungen und/oder Klebeeinrichtungen und so weiter handeln. Diese Maschinenkomponenten 3 und die Antriebe 4 sind als Montageeinheiten auf Trageplatten 9 je nach Bedarf zusammengefaßt und bilden jeweils gemeinsam ein Kleinmodul 10.

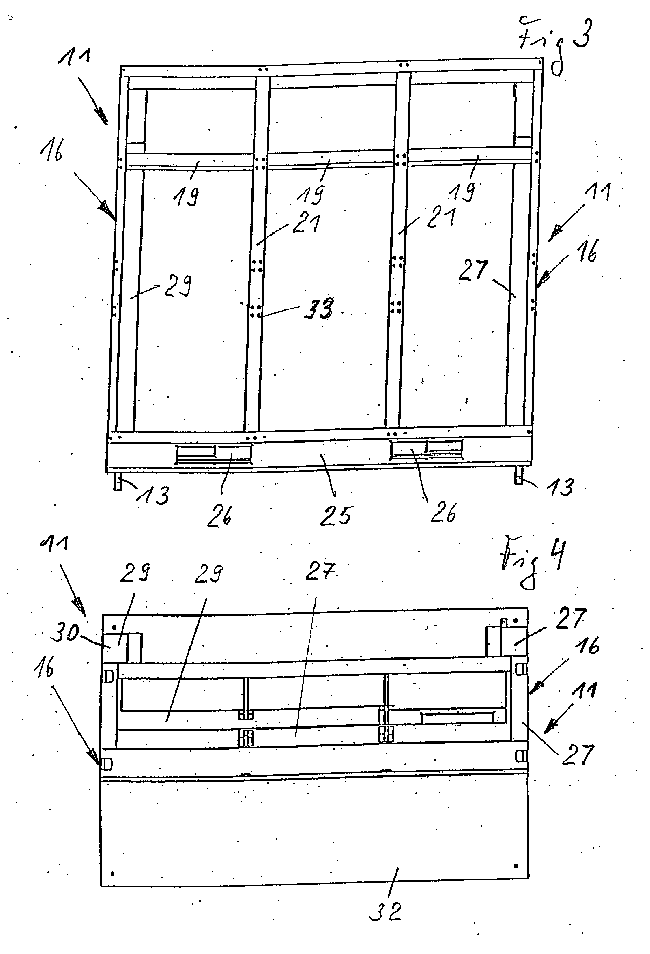

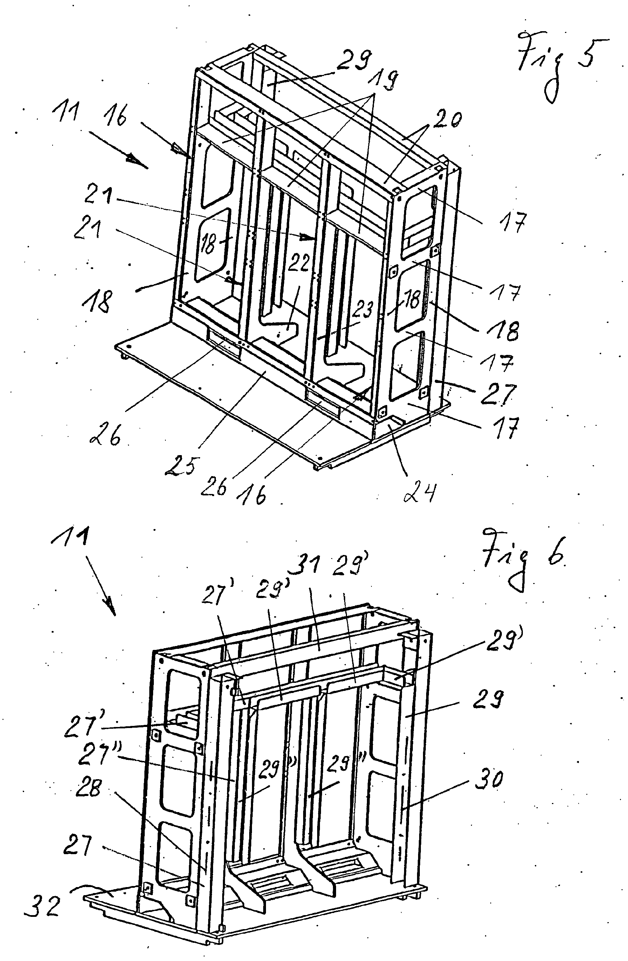

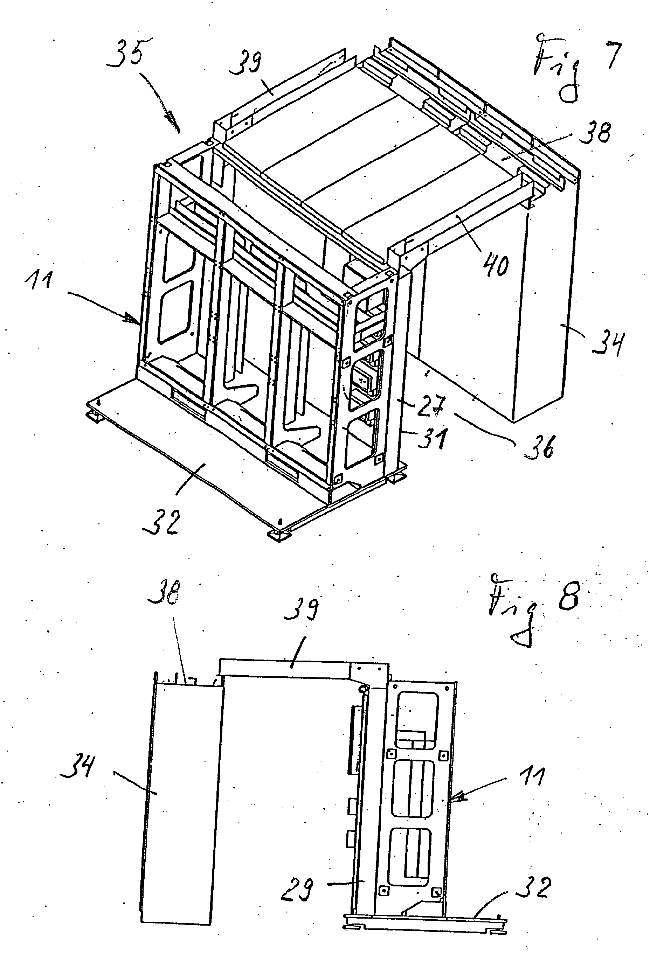

Als Träger für mehrere Kleinmodule 10 ist jeweils ein Montagegerüst 11 ( Fig. 5) vorgesehen und bildet zusammen mit den Kleinmodulen 10 jeweils ein Großmodul 12.

Die mit Kleinmodulen 10 bestückten Großmodule 12 sind zur Bildung der das Maschinengestell, die Maschinenkomponenten 3 und die Antriebe 4 umfassenden Vorrichtung 1 miteinander verbindbar. Dazu werden die Großmodule 12 miteinander verschraubt. Mit Hilfe einstellbarer Füße 13 lassen sie sich zuvor auf einem Boden 14 ausrichten ( Fig. 1).

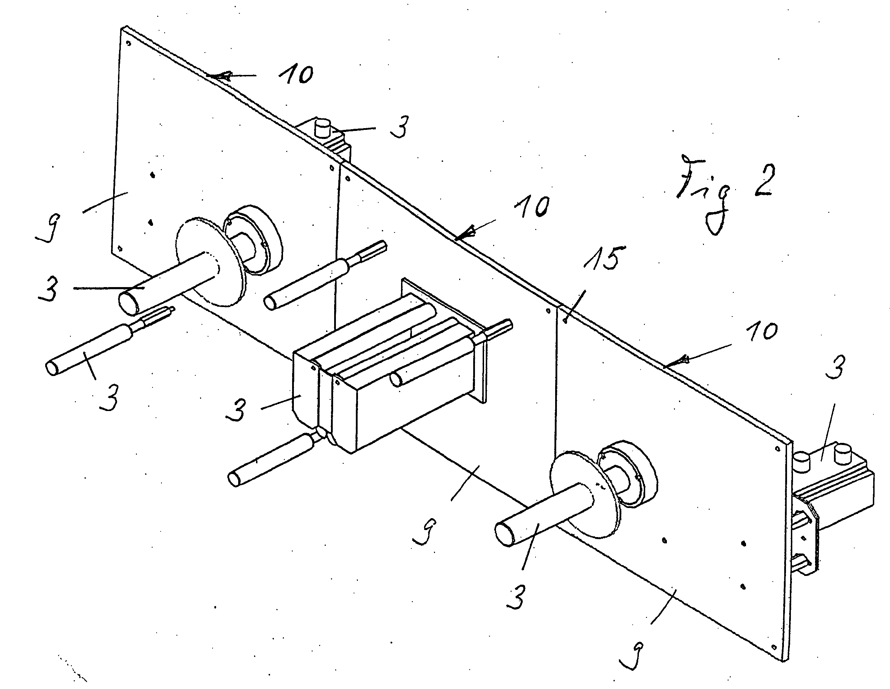

Die Befestigung der Trageplatten 9 der Kleinmodule 10 an den Montagegerüsten 11 der Großmodule 12 erfolgt mit Hilfe von Schrauben und Befestigungsöffnungen 15 in den Trageplatten 9 (Fig. 2).

Das Montagegerüst 11 umfaßt gemäß den Darstellungen in den Fig. 3 bis 6 quer zur Maschinenlängsrichtung stehende Rahmenelemente 16 mit kurzen, horizontalen Streben 17 und mit langen, vertikal angeordneten Stützen 18 sowie diese in Maschinenlängsrichtung verbindende Längsstreben 19 und 20. Ferner ist mindestens ein L-förmiges, quer zur Maschinenlängsrichtung stehendes Rahmenteil 21 vorgesehen und weist einen bodenseitig angeordneten kurzen Schenkel 22 und eine vertikal stehende Stütze 23 auf.

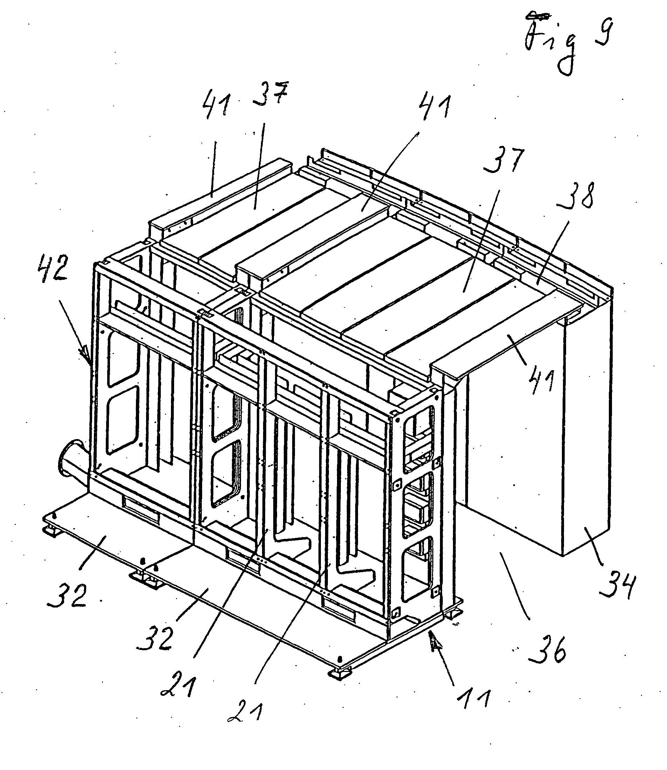

Gemäß dem in den Fig. 5 und 6 dargestellten Ausführungsbeispiel befinden sich zwei Rahmenteile 21 zwischen den die Enden des Montagegerüstes 11 bildenden Rahmenelementen 16.

Die rechteckigen Rahmenelemente 16 und die L-förmigen Rahmenteile 21 sind jeweils einstückig.

Ferner weisen die rechteckigen Rahmenelemente 16 und die L-förmigen Rahmenteile 21 jeweils bodenseitig eine Ausnehmung 24 für einen Luft führenden Kanal 25 auf. Der Luft führende Kanal 25 ist an seinen Enden offen und weist darüber hinaus auch zwischen seinen Enden zusätzliche Öffnungen 26 auf.

Der Luft führende Kanal 25 ist in das Montagegerüst 11 als ein die Festigkeit und Steifigkeit erhöhendes Element in gleicher Weise integriert wie weitere, horizontal und vertikal angeordnete Kabelkanäle 27 und 29

Das Montagegerüst 11 des Großmoduls 12 weist horizontal und vertikal angeordnete Kabelkanäle 27 für elektrische Steuerleitungen 28 und getrennt davon horizontal und vertikal angeordnete Kabelkanäle 29 für zur Kraftversorgung dienende elektrische Leitungen 30 auf. Sowohl die Kabelkanäle 27 als auch die Kabelkanäle 29 bestehen aus Kabelkanalstükken und sind an mehreren Stellen gut zugänglich.

Auf der Rückseite 31 des Montagegerüstes 11 erstreckt sich an einem Ende sowie fluchtend mit dem einen Rahmenelement 16 ein vertikal stehender Kabelkanal 27 und am anderen Ende erstreckt sich ebenfalls ein vertikal stehender Kabelkanal 29 von einer Bodenplatte 32 bis zum oberen Ende des Montagegerüstes 11. Etwa im oberen Viertel sind die horizontal liegenden Kabelkanalstücke 27`und 29`angeordent, von denen sich ebenfalls vertikal stehende Kabelkanalstücke 27`` und 29`` nach unten erstrecken.

Die Kabelkanäle 27 und 29 und entsprechend ihre Kabelkanalstücke sind derart gestaltet, daß sie zugleich eine tragende Funktion besitzen.

Der Luft führende Kanal 25 und in gleicher Weise der für Steuerleitungen 28 bestimmte Kabelkanal 27 und der für elektrische Leitungen zur Kraftversorgung bestimmte Kabelkanal 29 bzw. ihre Kabelkanalstücke bestehen wegen ihrer zugleich tragenden Funktion aus Stahl. Sie weisen eine Materialstärke auf, die etwa um 2,5 mm größer ist als die Materialstärke derartiger Kanäle ohne tragende Funktion.

Sowohl die außen befindlichen Rahmenelemente 16 als auch die dazwischen angeordneten Rahmenteile 21 weisen Befestigungsöffnungen 33 an definierten Stellen zum Befestigen der Trageplatten 9 der Kleinmodule 10 mit Hilfe von in den Fig. nicht dargestellten Schrauben auf.

Schließlich bildet ein im Abstand von dem Montagegerüst 11 sowie auf dessen Rückseite 31 angeordneter Schaltschrank 34 zusammen mit dem Montagegerüst 11 eine Baueinheit 35 ( Fig. 7 ). Der Abstand zwischen dem Montagegerüst 11 und dem parallel stehenden Schaltschrank 34 kann derart bemessen sein, daß sich ein Gang 36 zwischen diesen befindet. Ferner verbinden die Festigkeit und Stabilität erhöhende Dekkenelemente 37 oben die Rückseite 31 des Montagegerüstes 11 mit der Abdeckung 38 des Schaltschrankes 34. Zugleich überbrücken Kabelkanäle 39 und 40 den Gang 36 und verbinden die Kabelkanäle 27 und 29 mit entsprechenden Eingangsstellen an dem bzw. den Schaltschränken 34. Auch die Kabelkanäle 39 und 40 besitzen eine die Festigkeit und Tragfähigkeit des Montagegerüstes beziehungsweise des Großmodules 12 erhöhende Funktion.

Gemäß dem in Fig. 9 dargestellten Ausführungsbeispiel sind die im Querschnitt U-förmigen Kabelkanäle 39 und 40 mit Hilfe von Platten 41 abgedeckt.

Ferner steht gemäß Fig. 9 neben einem zwei L-förmige Rahmenteile 21 aufweisenden Montagegerüst 11 ein weiteres Montagegerüst 42 ohne L-förmige Rahmenteile 21. Die Erfindung ist daher nicht auf Großmodule 12 mit Montagegerüsten 11 beschränkt, die jeweils zwei Rahmenteile 21 aufweisen.

Grundsätzlich versteht es sich darüber hinaus, daß für die Trageplatten 9 und auch für Platzhalter ― Platten 43 ohne Tragefunktion jeweils Rastermaße vorgesehen sind, damit sie sich in sinnvoller und zweckmäßiger Weise zu Großmodulen 12 zusammenfassen lassen. Diese Rastermaße für Trage- bzw. für Platzhalterplatten betragen
a) 1200 mm x 700 mm
b) 1200 mm x 500 mm
c) 1200 mm x 230 mm
d) 800 mm x 700 mm
e) 800 mm x 500 mm
f) 800 mm x 230 mm,
wobei die besagten Abmessungen besonders sinnvoll und zweckmäßig sind.

Das Maschinengestell der Vorrichtung 1 besteht aus der Summe aller miteinander verbundener Montagegerüste 11 und Trageplatten 9 sowie Platzhalterplatten 43.

Von den Maschinenkomponenten 3 und Antrieben 4 auf den Trageplatten 9 führen elektrische Steuer- und Regelungsleitungen zu den jeweiligen Kabelkanalstücken beziehungsweise Kabelkanälen 27 und werden dort jeweils individuell verlegt und an den Enden der Kanäle mit Verbindungselementen gegebenenfalls derart versehen, daß die Montagegerüste als Großmodule schließlich zusammengestellt und schnell miteinander verbunden werden können. Die Anordnung der Kabelkanäle 27 und 29 ist dabei derart gewählt, daß sie sowohl eine übersichtliche und gut gegliederte sowie geordnete Anordnung der Leitungen ermöglichen als auch die Festigkeit und Stabilität des Großmodules 12 beziehungsweise seines Montagegerüstes 11 erhöhen beziehungsweise dazu beitragen.

## Patentansprüche

1. Vorrichtung zum Herstellen von Hygieneprodukten, die mehrere Stationen ( 2 ) mit zumindest teilweise unterschiedlichen Maschinenkomponenten ( 3 ) sowie mit größtenteils eigenen Antrieben ( 4 ) umfaßt,
wobei jeweils Maschinenkomponenten ( 3 ) und Antriebe ( 4 ) als Montageeinheiten auf Trageplatten ( 9 ) zusammengefaßt und in einem Maschinengestell befestigbar sind, das vertikale Stützen ( 18 ) und horizontal verbindende Streben ( 17, 19, 20 ) aufweist,
**dadurch gekennzeichnet, daß** die als Kleinmodule ( 10 ) vorgesehenen, aus Maschinenkomponenten ( 3 ) und Antrieben ( 4 ) sowie Trageplatten ( 9 ) bestehenden Montageeinheiten mindestens teilweise zu großen, ebenfalls Montageeinheiten bildenden Großmodulen ( 12 ) zusammengefaßt sind und
daß die mit Kleinmodulen ( 10 ) bestückten Großmodule ( 12 ) zur Bildung der das Maschinengestell, die Maschinenkomponenten ( 3 ) und ihren Antriebe ( 4 ) umfassenden Vorrichtung ( 1 ) miteinander verbindbar sind.

2. Vorrichtung nach Anspruch 1, **dadurch gekennzeichnet,**
**daß** die Großmodule ( 12 ) jeweils ein Montagegerüst ( 11) als Träger aufweisen.

3. Vorrichtung nach Anspruch 1 und 2 , **dadurch gekennzeichnet, daß** das Montagegerüst ( 11 ) des Großmoduls ( 12 ) quer zur Maschinenlängsrichtung stehende Rahmenelemente (16) mit kurzen, horizontalen Streben ( 17 ) und mit langen, vertikal angeordneten Stützen ( 18 ) sowie diese in Maschinenlängsrichtung verbindenden Längsstreben ( 19, 20 ) aufweist.

4. Vorrichtung nach mindestens einem der vorhergehenden Ansprüche, **dadurch gekennzeichnet,**
**daß** mindestens ein L-förmiges, quer zur Maschinenlängsrichtung stehendes Rahmenteil ( 21 ) zwischen rechteckigen Rahmenelementen ( 16 ) vorgesehen ist und bodenseitig angeordneten, kurzen Schenkel ( 22) und eine vertikal stehende Stütze ( 23 ) aufweist.

5. Vorrichtungen nach mindestens einem der vorhergehenden Ansprüche, **dadurch gekennzeichnet,**
**daß** die rechteckigen Rahmenelemente ( 16 ) und die L-förmigen Rahmenteile ( 21 ) jeweils einstückig sind.

6. Vorrichtung nach mindestens einem der vorhergehenden Ansprüche, **dadurch gekennzeichnet,**
**daß** die rechteckigen Rahmenelemente ( 16 ) und die L-förmigen Rahmenteile ( 21 ) jeweils bodenseitig eine Ausnehmung ( 24 ) für einen Luft führenden Kanal ( 25 ) aufweisen.

7. Vorrichtung nach mindestens einem der vorhergehenden Ansprüche, **dadurch gekennzeichnet,**
**daß** das Montagegerüst ( 11 ) des Großmoduls ( 12 ) horizontal und vertikal angeordnete Kabelkanäle ( 27, 29 ) getrennt für Steuerleitungen ( 28 ) und für zur Kraftversorgung dienende elektrische Leitungen ( 30 ) aufweist.

8. Vorrichtung nach mindestens einem der vorhergehenden Ansprüche, **dadurch gekennzeichnet,**
**daß** die Kabelkanäle ( 27, 29 ) einschließlich deren Kabelkanalstücken derart gestaltet sind, daß sie zugleich eine tragende Funktion besitzen.

9. Vorrichtung nach mindestens einem der vorhergehenden Ansprüche, **dadurch gekennzeichnet,**
**daß** der Luft führende, in Maschinenlängsrichtung angeordnete Kanal ( 25 ) eine zugleich tragende Funktion besitzt.

10. Vorrichtung nach mindestens einem der vorhergehenden Ansprüche, **dadurch gekennzeichnet,**
**daß** der Luft führende Kanal ( 25 ) und/oder die Kabelkanäle ( 27, 29 ) einschließlich ihrer Kabelkanalstücke mit jeweils zugleich tragender Funktion aus Stahl bestehen und eine Materialstärke aufweisen, die etwa um 2,5 mm größer ist als die Materialstärke derartiger Kanäle ohne tragende Funktion.

11. Vorrichtungen nach mindestens einem der vorhergehenden Ansprüche, **dadurch gekennzeichnet,**
**daß** Schaltschränke ( 34 ) im Abstand sowie parallel zu den Montagegerüsten ( 11/Großmodulen 12 ) angeordnet und mit diesen verbunden sind.

12. Vorrichtungen nach mindestens einem der vorhergehenden Ansprüche, **dadurch gekennzeichnet,**
**daß** Deckenplatten ( 37 ) und im Querschnitt U-förmige Kabelkanäle ( 39, 40 ) die Großmodule ( 12 ) und die Schaltschränke ( 34 ) verbinden.

13. Vorrichtungen nach mindestens einem der vorhergehenden Ansprüche, **dadurch gekennzeichnet,**
**daß** Trageplatten ( 9) mit tragender Funktion für Maschinenkomponenten ( 3 ) und ihre Antriebe ( 4 ) und/oder Trageplatten als Platzhalter - Platten ( 43 ) ohne Tragefunktion vorgesehen sind.

14. Vorrichtungen nach mindestens einem der vorhergehenden Ansprüche, **gekennzeichnet durch**,
folgende Rastermaße für Trage- bzw. Platzhalterplatten (9,43):
a) 1200 mm x 700 mm
b) 1200 mm x 500 mm
c) 1200 mm x 230 mm
d) 800 mm x 700 mm
e) 800 mm x 500 mm
f) 800 mm x 230 mm.
